# EUROPEAN PATENT SPECIFICATION

(11) **EP 0 767 796 B1**
(45) Date of publication and mention of the grant of the patent: **26.09.2001**
(21) Application number: 94917388.4
(22) Date of filing: 16.05.1994
(51) Int. Cl.: C12N 15/19, A61K 38/19, C07K 14/52

(54) **CHEMOTACTIC PROTEIN**
CHEMOTAKTISCHES PROTEIN
PROTEINE CHIMIOTACTIQUE

(43) Date of publication of application: 16.04.1997
(60) Divisional application: 01100052.8
(73) Proprietor: HUMAN GENOME SCIENCES, INC., Rockville, MD 20850-3338 (US)
(72) Inventor: LI, Haodong, Gaithersburg MD 20878 (US); RUBEN, Steven M., Olney, MD 20832 (US); SUTTON, Granger, G., III, Columbia, MD 21045 (US)
(74) Representative: VOSSIUS & PARTNER
(86) International application number: US9405384
(87) International publication number: WO9531467

(56) References cited:
- EP-A- 0 488 900
- BIOCHEMICAL AND BIOPHYSICAL RESEARCH COMMUNICATIONS, Volume 167, Number 3, issued 30 March 1990, DECOCK et al., "Identification of the Monocyte Chemotactic Protein from Human Osteosarcoma Cells and Monocytes: Detection of a Novel N-Terminally Processed Form", pages 904-909.
- BIOCHEMICAL AND BIOPHYSICAL RESEARCH COMMUNICATIONS, Volume 169, Number 2, issued 15 June 1990, SHYY et al., "Structure of Human Monocyte Chemotactic Protein Gene and Its Regulation by TPA", pages 346-351.
- BIOCHEMICAL AND BIOPHYSICAL RESEARCH COMMUNICATIONS, Volume 159, Number 1, issued 28 February 1989, FURUTANI et al., "Cloning and Sequencing of the cDNA for Human Monocyte Chemotactic and Activating Factor (MCAF)", pages 249-255.
- BIOCHEMICAL AND BIOPHYSICAL RESEARCH COMMUNICATIONS, Volume 174, Number 2, issued 31 January 1991, YOSHIMURA et al., "Molecular Cloning of Rat Monocyte Chemoattractant Protein-1 (MCP-1) and Its Expression in Rat Spleen Cells and Tumor Cell Lines", pages 504-509.
- INTERNATIONAL JOURNAL OF CANCER, Volume 45, issued 1990, BOTTAZZI et al., "A Chemoattractant Expressed in Human Sarcoma Cells (Tumor-Derived Chemotactic Factor, TDCF) is Identical to Monocyte Chemoattractant Protein-1/Monocyte Chemotactic and Activating Factor (MCP-1/MCAF)", pages 795-797.
- MOLECULAR AND CELLULAR BIOLOGY, Volume 9, Number 11, issued November 1989, ROLLINS et al., "The Human Homolog of the JE Gene Encodes a Monocyte Secretory Protein", pages 4687-4695.
- THE JOURNAL OF IMMUNOLOGY, Volume 146, Number 10, issued 15 May 1991, YOSHIMURA et al., "Neutrophil Attractant/Activation Protein-1 and Monocyte Chemoattractant Protein-1 in Rabbit", pages 3483-3488.
- DNA AND CELL BIOLOGY, Volume 10, Number 9, issued 1991, WEMPE et al., "Gene Expression and cDNA Cloning Identified a Major Basic Protein Constituent of Bovine Seminal Plasma as Bovine Monocyte-Chemoattractant Protein-1 (MCP-1)", pages 671-679.
- PROTEIN ENGINEERING, Volume 4, Number 3, issued 1991, GRONENBORN et al., "Modeling the Three-Dimensional Structure of the Monocyte Chemo-attractant and Activating Protein MCAF/MCP-1 on the Basis of the Solution Structure of Interleukin-8", pages 263-269.
- PROC. NATL. ACAD. SCI. U.S.A., Volume 86, issued March 1989, ROBINSON et al., "Complete Amino Acid Sequence of a Human Monocyte Chemoattractant, a Putative Mediator of Cellular Immune Reactions", pages 1850-1854.
- FEBS LETTERS, Volume 244, Number 2, issued February 1989, YOSHIMURA et al., "Human Monocyte Chemoattractant Protein-1 (MCP-1): Full-Length cDNA Cloning, Expression in Mitogen-Stimulated Blood Mononuclear Leukocytes and Sequence Similarity to Mouse Competence Gene JE", pages 487-493.
- EUROPEAN JOURNAL OF BIOCHEMISTRY, Volume 199, issued 1991, VAN DAMME et al., "Production and Identification of Natural Monocyte Chemotactic Protein from Virally Infected Murine Fibroblasts", pages 223-229.
- PROC. NATL. ACAD. SCI. U.S.A., Volume 85, issued June 1988, ROLLINS et al., "Cloning and Expression of JE, a Gene Inducible by Platelet-Derived Growth Factor and Whose Product has Cytokine-Like Properties", pages 3738-3742.

### SEQUENCE LISTING

(1) GENERAL INFORMATION:
   (i) APPLICANT: LI, ET AL.
   (ii) TITLE OF INVENTION: Chemotactic Protein
   (iii) NUMBER OF SEQUENCES: 2
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: Submitted herewith
      (C) CLASSIFICATION:
   (vii) PRIOR APPLICATION DATA
      (A) APPLICATION NUMBER:
      (B) FILING DATE:
   (viii) ATTORNEY/AGENT INFORMATION:
      (A) NAME: FERRARO, GREGORY D.
      (B) REGISTRATION NUMBER: 36,134
      (C) REFERENCE/DOCKET NUMBER: 325800-160
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 360 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   ATGGCAGGCC TGATGACCAT AGTAACCAGC CTTCTGTTCC TTGGTGTCTG TGCCCACCAC
   60
   ATCATCCCTA CGGGCTCTGT GGTCATACCC TCTCCCTGCT GCATGTTCTT TGTTTCCAAG
   120
   AGAATTCCTG AGAACCGAGT GGTCAGCTAC CAGCTGTCCA GCAGGAGCAC ATGCCTCAAG
   180
   GGAGGAGTGA TCTTCACCAC CAAGAAGGGC CAGCAGTTCT GTGGCGACCC CAAGCAGGAG
   240
   TGGGTCCAGA GGTACATGAA GAACCTGGAC GCCAAGCAGA AGAAGGCTTC CCCTAGGGCC
   300
   AGGGCAGTGG CTGTCAAGGG CCCTGTCCAG AGATATCCTG GCAACCAAAC CACCTGCTAA
   360
(2) INFORMATION FOR SEQ ID NO:2:
   (i) SEQUENCE CHARACTERISTICS
      (A) LENGTH: 119 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2:

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention is chemotactic protein (CP). The invention also relates to inhibiting the action of such polypeptides.

There are three forms of monocyte chemotactic protein, namely, MCP-1, MCP-2 and MCP-3. All of these proteins have been structurally and functionally characterized and have also been cloned and expressed. MCP-1 and MCP-2 have the ability to attract leukocytes (monocytes, and leukocytes), while MCP-3 also attracts eosinophils and T lymphocytes (Dahinderi, E. et al., J. Exp. Med., 179:751-756 (1994)).

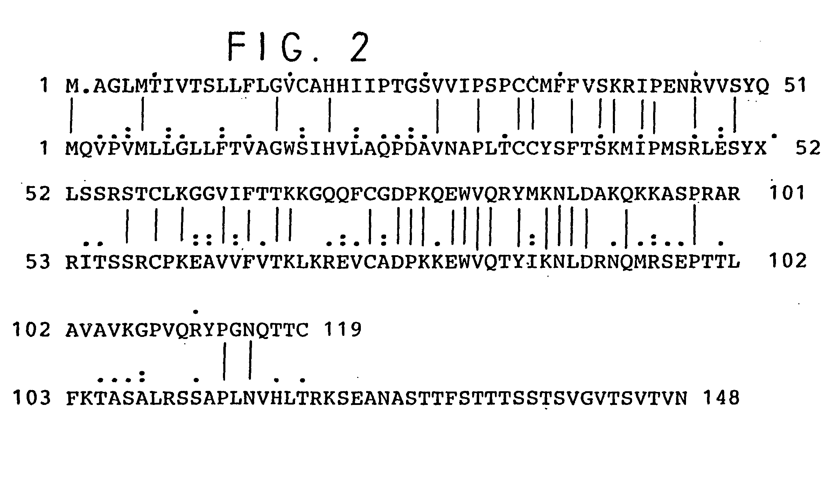

Initially, human monocyte-specific attracting factor, was purified from a glioma cell line and a monocytic cell line. Matsushima, K. et al, J. Exp. Med., 169:1485-1490 (1989). This factor was originally designated glioma-derived chemotactic factor (GDCF) and monocyte chemotactic and activating factor (MCAF) by Matsushima, et al. This factor is now referred to as MCP-1. Subsequent cloning of the cDNA for MCP-1 showed it to be highly similar to the murine JE gene. The JE gene could be massively induced in murine fibroblasts by platelet-derived growth factor. Cochran, B.H., et al, Cell 33:939-947 (1983). Murine JE is highly similar to MCP-1. The MCP-1 protein is 62% identical to murine JE in a region of 68 shared N-terminal residues. It is widely accepted that JE and MCP-1 are species homologs. The polypeptide of the present invention, CP is both structurally and functionally related to MCP-1 and the murine JE protein, see Figure 2.

A method of suppressing tumor. formation in a vertebrate by administering JE/MCP-1 has been disclosed in PCT application WO-92/20372, along with methods of treating localized complications of malignancies and methods of combatting parasitic infection by administering JE/MCP-1. Expression of the JE/MCP-1 protein in malignant cells was found to suppress the cells ability to form tumors *in vivo.*

Human MCP-1 is a basic peptide of 76 amino acids with a predicted molecular mass of 8,700 daltons. MCP-1 is inducibly expressed mainly in monocytes, endothelial cells and fibroblasts. Leonard, E.J. and Yoshimura, T., Immunol. Today, 11:97-101 (1990). The factors which induce this expression is IL-1, TNF or lipopolysaccharide treatment.

Other properties of MCP-1 include the ability to strongly activate mature human basophils in a pertussis toxin-sensitive manner. MCP-1 is a cytokine capable of directly inducing histamine release by basophils, (Bischoff, S.C. et al., J. Exp. Med., 175:1271-1275 (1992)). Furthermore, MCP-1 promotes the formation of leukotriene C4 by basophils pretreated with Interleukin 3, Interleukin 5, or granulocyte/macrophage colony-stimulating factor. MCP-1 induced basophil mediator release may play an important role in allergic inflammation and other pathologies expressing MCP-1.

Clones having a nucleotide sequence encoding a human monocyte chemotactic and activating factor (MCAF) reveal the primary structure of the MCAF polypeptide to be composed of a putative signal peptide sequence of 23 amino acid residues and a mature MCAF sequence of 76 amino acid residues. Furutani, Y.H., et al, Biochem. Biophys. Res. Commu., 159:249-55 (1989). The complete amino acid sequence of human glioma-derived monocyte chemotactic factor (GDCF-2) has also been determined. This peptide attracts human monocytes but not neutrophils. It was established that GDCF-2 comprises 76 amino acid residues. The peptide chain contains 4 half-cysteines, at positions 11, 12, 36 and 52, which create a pair of loops, clustered at the disulfide bridges. Further, the MCP-1 gene has been designated to human chromosome 17. Mehrabian, M.R., et al, Genomics, 9:200-3 (1991). Furthermore, an analogue of MCP-1 has been reported in EP-A-488900. Certain data suggests that a potential role for MCP-1 is mediating monocytic infiltration of the artery wall. Monocytes appear to be central to atherogenesis both as the progenitors of foam cells and as a potential source of growth factors mediating intimal hyperplasia. Nelken, N.A., et al, J. Clin. Invest., 88:1121-7 (1991). It has also been found that synovial production of MCP-1 may play an important role in the recruitment of mononuclear phagocytes during inflammation associated with rheumatoid arthritis and that synovial tissue macrophages are the dominant source of this cytokine. MCP-1 levels were found to be significantly higher in synovial fluid from rheumatoid arthritis patients compared to synovial fluid from osteoarthritis patients or from patients with other arthritides. Koch, A.E., et al, J. Clin. Invest., 90:772-9 (1992).

MCP-2 and MCP-3 are classified in a subfamily of proinflammatory proteins and are functionally related to MCP-1 because they specifically attract monocytes, but not neutrophils. Van Damme, J., et al, J. Exp. Med., 176:59-65 (1992). MCP-3 shows 71% and 58% amino acid homology to MCP-1 and MCP-2 respectively. MCP-3 is an inflammatory cytokine that regulates macrophage functions.

In accordance with one aspect of the present invention, there is provided a novel mature polypeptide which is, as well as fragments thereof. The polypeptide of the present invention is of human origin.

In accordance with another aspect of the present invention, there are provided polynucleotides (DNA or RNA) which encode such polypeptides.

In accordance with yet a further aspect of the present invention, there is provided a process for producing such polypeptide by recombinant techniques.

In accordance with yet a further aspect of the present invention, there is provided a process for utilizing such polypeptide, or polynucleotide encoding such polypeptide for therapeutic purposes, for example, to treat tumors, to promote wound healing, to combat parasitic infection and to regulate hematopoiesis.

In accordance with yet a further aspect of the present invention, there is provided an antibody against such polypeptides.

In accordance with yet another aspect of the present invention, there are provided methods for the identification of antagonist/inhibitors to such polypeptides, which may be used to inhibit the action of such polypeptides for therapeutic purposes, for example, to treat rheumatoid arthritis, lung inflammation, allergy, infectious diseases and to prevent inflammation and atherosclerosis.

These and other aspects of the present invention should be apparent to those skilled in the art from the teachings herein.

The following drawings are illustrative of embodiments of the invention and are not meant to limit the scope of the invention as encompassed by the claims.

FIG. 1 depicts the cDNA sequence and corresponding deduced amino acid sequence of CP. The 119 amino acid sequence shown is the full length protein, with approximately the first 22 amino acids representing a leader sequence such that the mature form of the protein is 97 amino acids in length. The standard one letter abbreviation for amino acids is used.

FIG. 2 illustrates the cDNA sequence homology between CP and the murine JE protein. The top sequence in each three segments is CP and the bottom sequence is murine JE protein.

FIG. 3 shows the results of a Northern blot analysis of the mRNA transcript for CP in human cells.

Figure 4 shows the banding pattern of human CP following bacterial expression and purification.

Figure 5 is a schematic representation of the pQE-9 vector.

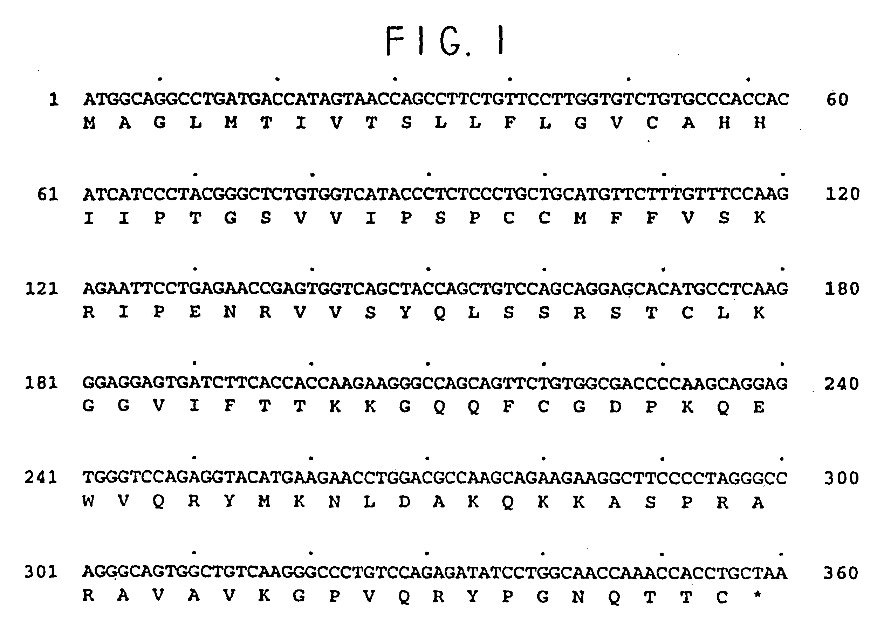

In accordance with an aspect of the present invention, there is provided an isolated nucleic acid (polynucleotide) which encodes for the mature polypeptide having the deduced amino acid sequence of Figure 1 or for the mature polypeptide encoded by the cDNA of the clone deposited as ATCC Deposit No. 75703 on March 10, 1994.

The polynucleotide of this invention was discovered from an activated monocyte cDNA library. It contains an open reading frame encoding a protein of approximately 119 amino acids in length of which the first 22 amino residues comprise a putative leader sequence. The mature protein therefore is predicted to be 97 amino acids in length. It is structurally related to mouse monocyte chemotactic protein (MCP-1 or JE), showing 27% identity, and 56% similarity over the entire human MCP-1 protein sequence. The polypeptide contains all four cysteine residues that occur in all chemokines in a characteristic motif. The spacing between these cysteines is conserved compared with the murine MCP-1/JE which strongly suggests that the new gene is a chemokine.

The polynucleotide of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. The coding sequence which encodes the mature polypeptide may be identical to the coding sequence shown in Figure 1 or that of the deposited clone or may be a different coding sequence which coding sequence, as a result of the redundancy or degeneracy of the genetic code, encodes the same, mature polypeptide as the DNA of Figure 1 or the deposited cDNA.

The polynucleotide which encodes for the mature polypeptide of Figure 1 or for the mature polypeptide encoded by the deposited cDNA may include: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 or the polypeptide encoded by the cDNA of the deposited clone. The variant of the polynucleotide may be a naturally occurring allelic variant of the polynucleotide or a non-naturally occurring variant of the polynucleotide.

Thus, the present invention includes polynucleotides encoding the same mature polypeptide as shown in Figure 1 or the same mature polypeptide encoded by the cDNA of the deposited clone as well as variants of such polynucleotides which variants encode for a fragment, derivative or analog of the polypeptide of Figure 1 or the polypeptide encoded by the cDNA of the deposited clone. Such nucleotide variants include deletion variants, substitution variants and addition or insertion variants.

As hereinabove indicated, the polynucleotide may have a coding sequence which is a naturally occurring allelic variant of the coding sequence shown in Figure 1 or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotide of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexa-histidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 70% identity between the sequences. The present invention particularly relates to polynucleotides which hybridize under stringent conditions to the hereinabove-described polynucleotides . As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptide encoded by the cDNA of Figure 1 or the deposited cDNA.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to a CP polypeptide which has the deduced amino acid sequence of Figure 1 or which has the amino acid sequence encoded by the deposited cDNA, as well as fragments, analogs and derivatives of such polypeptide.

The terms "fragment," "derivative" and "analog" when referring to the polypeptide of Figure 1 or that encoded by the deposited cDNA, means a polypeptide which retains essentially the same biological function or activity as such polypeptide. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the CP genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila and Sf9; animal cells such as CHO, COS or Bowes melanoma; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pbs, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptide can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. It is preferred to have low concentrations (approximately 0.15-5 mM) of calcium ion present during purification. (Price et al., J. Biol. Chem., 244:917 (1969)). Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The polypeptides of the present invention, in particular CP, may be used for the promotion of wound healing. Since CP is a chemokine, it is a chemo-attractant for leukocytes (such as basophils, etc.); therefore, it causes infiltration of target immune cells to a wound area.

The CP polypeptides may also be used as an antitumor treatment and for treating localized complications of a malignancy, such as pleural effusions or ascites.

The presence of MCPs *in vivo* is accompanied by a local increase in the presence of eosinophils which have the distinctive function of killing the larvae of parasites that invade tissues, as in schistosomiasis, trichinosis and ascariasis. Therefore, CP may be used for combatting parasitic infections.

CP polypeptides may also play a role in the regulation of hematopoiesis, by regulating various hematopoietic progenitor cell activation and differentiation.

The polypeptides may also be employed in accordance with the present invention by expression of such polypeptides *in vivo,* which is often referred to as "gene therapy."

For example, cells from a patient may be engineered with a polynucleotide (DNA or RNA) encoding an CP polypeptide *ex vivo,* with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of an CP polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo.* These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells *in vivo* after combination with a suitable delivery vehicle.

The polypeptides of the present invention may be employed in combination with a suitable pharmaceutical carrier. Such compositions comprise a therapeutically effective amount of the polypeptide, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

The pharmaceutical compositions may be administered in a convenient manner such as by the topical, intravenous, intraperitoneal, intramuscular, subcutaneous, intranasal or intradermal routes. CP is administered in an amount which is effective for treating and/or prophylaxis of the specific indication. The amounts and dosage regimens of CP administered to a subject will depend on a number of factors such as the mode of administration, the nature of the condition being treated and the judgment of the prescribing physician. In general, the CP will be administered in an amount of at least about 10 *µ*g/kg body weight and in most cases they will be administered in an amount not in excess of about 8 mg/Kg body weight per day. In most cases, the dosage is from about 10 *µ*g/kg to about 1 mg/kg body weight daily, taking into account the routes of administration, symptoms, etc.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the cDNA is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clones to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the EST was derived, and the longer the better. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

Comparison of affected and unaffected individuals generally involves first looking for structural alterations in the chromosomes, such as deletions or translocations that are visible from chromosome spreads or detectable using PCR based on that cDNA sequence. Ultimately, complete sequencing of genes from several individuals is required to confirm the presence of a mutation and to distinguish mutations from polymorphisms.

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention.

The present invention also relates to a diagnostic assay for detecting the level of CP both quantitatively and qualitatively. Such assays are well known in the art and include an ELISA assay and the radioimmunoassay. The levels of CP detected in the assay can be useful for the elucidation of the significance of CP in various diseases and for the diagnosis of diseases in which CP may play a role.

This invention provides a method for identification of CP receptors. The gene encoding an CP receptor can be identified by expression cloning. Briefly, polyadenylated RNA is prepared from a cell responsive to CP and a cDNA library created from this RNA is divided into pools and used to transfect COS cells or other cells that are not responsive to CP. Transfected cells which are grown on glass slides are exposed to labeled CP. CP can be labeled by a variety of means including iodidation or inclusion of a recognition site for a site-specific protein kinase. Following fixation and incubation, the slides are subjected to autoradiographic analysis. Positive pools are identified and sub-pools are prepared and retransfected using an iterative sub-pooling and rescreening process, eventually yielding a single clone that encodes the putative receptor. As an alternative approach for receptor identification, labeled CP can be photoaffinity linked with cell membrane or extract preparations that express an CP receptor molecule. Cross-linked material is resolved by PAGE and exposed to x-ray film. The labeled complex containing the CP receptor can be excised, resolved into peptide fragments, and subjected to protein microsequencing. The amino acid sequence obtained from microsequencing would be used to design a set of generate oligonucleotide probes to screen a cDNA library to identify a gene encoding the putative receptor.

This invention also provides a method of screening drugs to identify those which enhance (agonists) or block (antagonists) interaction of CP to its receptor. An agonist increases the biological functions of CP, while an antagonist reduces or eliminates such functions. As an example, a mammalian cell or membrane preparation expressing an CP receptor would be incubated with labeled CP in the presence of drug. The ability of drug to enhance or block this interaction could then be measured. Alternatively, the response of a known second messenger system following interaction of CP and its receptor would be measured compared in the presence or absence of drug. Such second messenger systems include but are not limited to, cAMP guanylate cyclase, ion channels or phosphoinositide hydrolysis.

The present invention is also directed to a method of identifying antagonist/inhibitor molecules to the polypeptides of the present invention. Antagonists include negative dominant mutants of CP. CP is a tetrameric polypeptide wherein one mutated unit will cause the entire polypeptide to be non-functional. A negative dominant mutant of CP binds to the CP receptor but fails to activate cells (leukocytes CP and monocytes to which it binds. An assay to detect negative dominant mutants of CP is an *in vitro* chemotaxis assay wherein a multiwell chemotaxis chamber equipped with polyvinylpyrrolidone-free polycarbonate membranes is used to measure the chemoattractant ability of CP for leukocytes in the presence and absence of potential antagonist/inhibitor or agonist molecules.

An example of an inhibitor is an antisense DNA or RNA construct. Antisense technology can be used to control gene expression through triple-helix formation or antisense DNA or RNA, both of which methods are based on binding of a polynucleotide to DNA or RNA. For example, the 5' coding portion or the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of CP. The antisense RNA. oligonucleotide hybridizes to the mRNA *in vivo* and blocks translation of the mRNA molecule into the CP (antisense - Okano, J. Neurochem., 56:560 (1991); Oligodeoxynucleotides as Antisense Inhibitors of Gene Expression, CRC Press, Boca Raton, FL (1988)). Alternatively, antisense RNA and DNA may be delivered to cells such that they are expressed *in vivo* to inhibit production of CP.

Another example of an antagonist identifiable by the method of the present invention is a peptide derivative of CP which are naturally or synthetically modified analogs of CP that have lost biological function yet still recognize and bind to receptors thereby effectively blocking the receptors.

The antagonist/inhibitors may be used to treat inflammation by preventing the attraction of monocytes to a wound or a site of trauma, and to regulate normal pulmonary macrophage populations, since acute and chronic inflammatory pulmonary diseases are associated with sequestration of mononuclear phagocytes in the lung. antagonist/inhibitors may be employed in a composition with a pharmaceutically acceptable carrier, e.g., as hereinabove described.

The present invention also relates to an assay for identifying potential antagonist/inhibitors specific to CP. An example of such an assay combines CP and a potential antagonist/inhibitor with membrane-bound CP receptors or recombinant CP receptors under appropriate conditions for a competitive inhibition assay. CP can be labeled, such as by radioactivity, such that the number of CP molecules bound to the receptor can determine the effectiveness of the potential antagonist/inhibitor.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 *µ*g of plasmid or DNA fragment is used with about 2 units of enzyme in about 20 *µ*l of buffer solution. For the purpose of isolating DNA fragments for plasmid construction, typically 5 to 50 *µ*g of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al.,* Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units to T4 DNA ligase ("ligase") per 0.5 *µ*g of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of CP

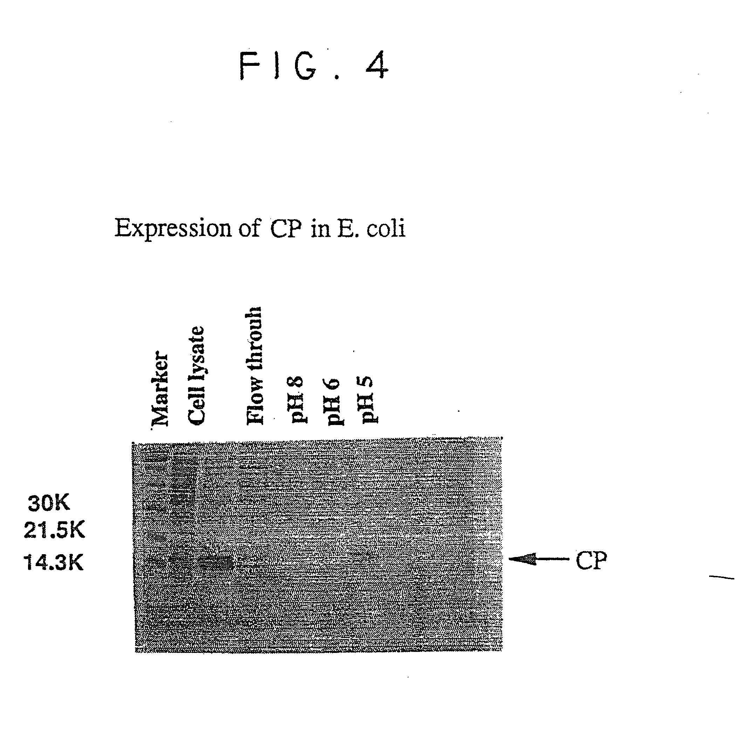

The DNA sequence encoding for CP ATCC # 75703, is initially amplified using PCR oligonucleotide primers corresponding to the 5' and 3' sequences of the processed CP (minus the signal peptide sequence) and the vector sequences 3' to the CP gene. Additional nucleotides corresponding to CP were added to the 5' and 3' sequences respectively. The 5' oligonucleotide primer has the sequence 5'-TCAGGATCCCCTACGGGCTCGTGGTC-3' contains a Bam H1 restriction enzyme site followed by 18 nucleotides of CP coding sequence starting from the presumed terminal amino acid of the processed protein codon. The 3' sequence 3'-CGCTCTAGAGTAAAACGACGGCCAGT-5' contains complementary sequences to the XbaI site and to a pBluescript SK- vector sequence located 3' to the CP DNA insert. The restriction enzyme sites correspond to the restriction enzyme sites on the bacterial expression vector pQE-9. (Qiagen, Inc. 9259 Eton Avenue, Chatsworth, CA, 91311). pQE-9 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-9 was then digested with Bam H1 and Xba I. The amplified sequences were ligated into pQE-9 and were inserted in frame with the sequence encoding for the histidine tag and the RBS. Figure 5 shows a schematic representation of this arrangement. The ligation mixture was then used to transform E. coli strain m15/rep4 available from Qiagen under the trademark M15/rep 4 by the procedure described in Sambrook, J. et al, Molecular Cloning: A Laboratory Manual, Cold Spring Laboratory Press, 1989. M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture is used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation. The cell pellet was solubilized in the chaotropic agent 6 Molar Guanidine HCl. After clarification, solubilized CP was purified from this solution by chromatography on a Nickel-Chelate column under conditions that allow for tight binding by proteins containing the 6-His tag. Hochuli, E. et al., J. Chromatography 411:177-184 (1984). CP (95% pure) was eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized). After incubation in this solution for 12 hours the protein was dialyzed to 10 mmolar sodium phosphate. Figure 4.

### Example 2

### Expression pattern of CP in human cells CP

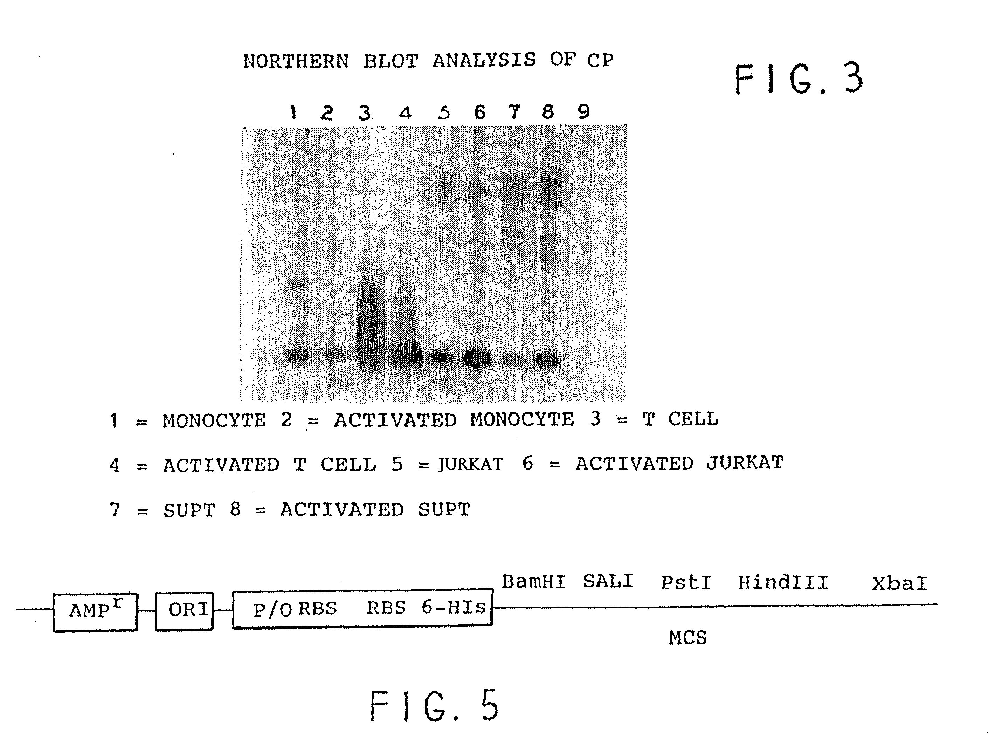

Northern blot analysis was carried out to examine the levels of expression of CP in human cells. Total cellular RNA samples were isolated with RNAzol™ B system (Biotecx Laboratories, Inc. 6023 South Loop East, Houston, TX 77033). About 10*µ*g of total RNA isolated from each human tissue specified was separated on 1% agarose gel and blotted onto a nylon filter. (Sambrook, Fritsch, and Maniatis, Molecular Cloning, Cold Spring Harbor Press, (1989)). The labeling reaction was done according to the Stratagene Prime-It kit with 50ng DNA fragment. The labeled DNA was purified with a Select-G-50 column. (5 Prime - 3 Prime, Inc. 5603 Arapahoe Road, Boulder, CO 80303). The filter was then hybridized with radioactive labeled full length CP gene at 1,000,000 cpm/ml in 0.5 M NaPO₄, pH 7.4 and 7% SDS overnight at 65°C. After wash twice at room temperature and twice at 60°C with 0.5 x SSC, 0.1% SDS, the filter was then exposed at -70°C overnight with an intensifying screen. The message RNA for CP is abundant in activated and unactivated T cells, monocytes and T cell lines. Figure 3.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in Figure 1;
(b) polynucleotides having the coding sequence as shown in Figure 1 coding for at least the mature form of the polypeptide;
(c) polynucleotides encoding the polypeptide having the amino acid sequence of at least the mature form of the polypeptide encoded by the cDNA contained in ATCC 75703;
(d) polynucleotides having the coding sequence of the cDNA contained in ATCC 75703 coding for at least the mature form of the polypeptide;
(e) polynucleotides encoding a fragment of a polypeptide encoded by a polynucleotide of any one of (a) to (d), said fragment being a chemoattractant for leukocytes;
(f) polynucleotides, the sequence of which is at least 70% identical to the sequence of a polynucleotide of any one of (a) to (d) and which encode a polypeptide which is a chemoattractant for leukocytes; and
(g) polynucleotides the sequence of which is at least 70% identical to the sequence of a polynucleotide of any one of (a) to (d) and the complementary strand of which hybridizes under stringent conditions to a polynucleotide as defined in any one of (a) to (d) and which encode a polypeptide which is a chemoattractant for leukocytes;
or the complementary strand of such a polynucleotide.

2. The polynucleotide of claim 1, wherein the encoded polypeptide or fragment thereof is a chemoattractant for basophils.

3. The polynucleotide of claim 1 or 2 which is DNA or RNA.

4. The DNA of claim 3 which is genomic DNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the vector of claim 5 or 6.

8. A process for producing polypeptide, which is a chemoattractant for leukocytes, comprising: culturing the host cell of claim 7 and recovering the polypeptide encoded by said polynucleotide from the culture.

9. A process for producing cells capable of expressing a polypeptide, which is a chemoattractant for leukocytes, comprising genetically engineering cells with the vector of claim 5 or 6.

10. A polypeptide comprising the amino acid sequence encoded by a polynucleotide of any one of claims 1 to 4 or obtainable by the process of claim 8.

11. The polypeptide of claim 10 which comprises an N-terminal methionine residue.

12. An antibody against the polypeptide of claim 10 or 11.

13. An antagonist/inhibitor against the polypeptide of claim 10 or 11, said antagonist/inhibitor being an antisense DNA or RNA construct.

14. A nucleic acid molecule which specifically hybridizes under stringent conditions to a polynucleotide of any one of claims 1 to 4.

15. A pharmaceutical composition comprising the polynucleotide of any one of claims 1 to 4, the nucleic acid molecule of claim 14, an antagonist/inhibitor of claim 13 or the polypeptide of claim 10 or 11 and optionally a pharmaceutically acceptable carrier.

16. A diagnostic composition comprising a polynucleotide of any one of claims 1 to 4, the nucleic acid molecule of claim 14 or the antibody of claim 12.

17. Use of a polypeptide of claim 10 or 11 or of a polynucleotide of any one of claims 1 to 4 for the preparation of a pharmaceutical composition for the promotion of wound healing, for the treatment of tumors, pleural effusions or ascites, for combating parasitic infections or for the regulation of hematopoiesis.

18. The use of claim 17, wherein the parasitic infection is schistosomiasis, trichinosis or ascariasis.

19. Use of the antagonist/inhibitor of claim 13 for the preparation of a pharmaceutical composition for the treatment of inflammation, rheumatoid arthritis, atherosclerosis, allergy or infectious diseases.

20. A method for identifying an antagonist/inhibitor or agonist/activator to the polypeptide of claim 10 comprising:
(a) contacting the polypeptide and a mammalian cell or a membrane preparation expressing a receptor of the polypeptide with a drug; and
(b) measuring whether the drug enhances or blocks the interaction of the polypeptide with its receptor or the response that results from such interaction.

## Patentansprüche

1. Polynucleotid ausgewählt aus der Gruppe bestehend aus
(a) Polynucleotiden, die zumindest die reife Form des Polypeptids codieren, das die abgeleitete Aminosäuresequenz wie in Figur 1 gezeigt aufweist;
(b) Polynucleotiden, die die codierende Sequenz wie in Figur 1 gezeigt aufweisen, die zumindest die reife Form des Polypeptids codiert;
(c) Polynucleotiden, die das Polypeptid codieren, das die Aminosäuresequenz von zumindest der reifen Form des Polypeptids aufweist, das von der in ATCC 75703 enthaltenen cDNA codiert wird;
(d) Polynucleotiden, die die codierende Sequenz der in ATCC 75703 enthaltenen cDNA aufweisen, die zumindest die reife Form des Polypeptids codiert;
(e) Polynucleotiden, die ein Fragment eines Polypeptids codieren, das von einem Polynucleotid gemäß einem der Teile (a) bis (d) codiert wird, wobei das Fragment ein chemotaktischer Lockstoff für Leukozyten ist;
(f) Polynucleotiden, deren Sequenz zumindest zu 70% identisch ist mit der Sequenz eines Polynucleotids gemäß einem der Teile (a) bis (d) und die ein Polypeptid codieren, das ein chemotaktischer Lockstoff für Leukozyten ist; und
(g) Polynucleotiden, deren Sequenz zumindest zu 70% identisch ist mit der Sequenz eines Polynucleotids gemäß einem der Teile (a) bis (d) und deren komplementärer Strang unter stringenten Bedingungen mit einem gemäß einem der Teile (a) bis (d) definierten Polynucleotid hybridisiert, und die ein Polypeptid codieren, das ein chemotaktischer Lockstoff für Leukozyten ist;
oder der komplementäre Strang eines solchen Polynucleotids.

2. Polynucleotid nach Anspruch 1, wobei das codierte Polypeptid oder ein Fragment davon ein chemotaktischer Lockstoff für Basophile ist.

3. Polynucleotid nach Anspruch 1 oder 2, das DNA oder RNA ist.

4. DNA nach Anspruch 3, die genomische DNA ist.

5. Vektor, enthaltend das Polynucleotid nach einem der Ansprüche 1 bis 4.

6. Vektor nach Anspruch 5, wobei das Polynucleotid funktionell verbunden ist mit Expressionskontrollsequenzen, die eine Expression in prokaryontischen oder eukaryontischen Wirtszellen erlauben.

7. Wirtszelle, die genetisch verändert ist mit dem Vektor nach Anspruch 5 oder 6.

8. Verfahren zur Herstellung eines Polypeptids, das ein chemotaktischer Lockstoff für Leukozyten ist, umfassend: Züchten der Wirtszelle nach Anspruch 7 und Gewinnen des Polypeptids, das von dem Polynucleotid codiert wird, aus der Kultur.

9. Verfahren zur Herstellung von Zellen, die in der Lage sind, ein Polypeptid zu exprimieren, das ein chemotaktischer Lockstoff für Leukozyten ist, umfassend das genetische Verändern von Zellen mit dem Vektor nach Anspruch 5 oder 6.

10. Polypeptid, umfassend die Aminosäuresequenz, die von einem Polynucleotid nach einem der Ansprüche 1 bis 4 codiert wird, oder erhältlich durch das Verfahren nach Anspruch 8.

11. Polypeptid nach Anspruch 10, das einen N-terminalen Methioninrest umfaßt.

12. Antikörper, der gerichtet ist gegen das Polypeptid nach Anspruch 10 oder 11.

13. Antagonist/Inhibitor, der gerichtet ist gegen das Polypeptid nach Anspruch 10 oder 11, wobei der Antagonist/Inhibitor ein Antisense-DNA- oder RNA-Konstrukt ist.

14. Nucleinsäuremolekül, das unter stringenten Bedingungen spezifisch mit einem Polynucleotid nach einem der Ansprüche 1 bis 4 hybridisiert.

15. Arzneimittel, umfassend das Polynucleotid nach einem der Ansprüche 1 bis 4, das Nucleinsäuremolekül nach Anspruch 14, den Antagonisten/Inhibitor nach Anspruch 13 oder das Polypeptid nach Anspruch 10 oder 11 und gegebenenfalls einen pharmazeutisch verträglichen Träger.

16. Diagnostische Zusammensetzung, umfassend ein Polynucleotid nach einem der Ansprüche 1 bis 4, das Nucleinsäuremolekül nach Anspruch 14 oder den Antikörper nach Anspruch 12.

17. Verwendung eines Polypeptids nach Anspruch 10 oder 11 oder eines Polynucleotids nach einem der Ansprüche 1 bis 4 zur Herstellung eines Arzneimittels zur Förderung von Wundheilung, zur Behandlung von Tumoren, Pleuraergüssen oder Ascites, zur Bekämpfung parasitischer Infektionen oder zur Regulation der Hematopoiese.

18. Verwendung nach Anspruch 17, wobei die parasitische Infektion Schistosomiasis, Trichinosis oder Askariasis ist.

19. Verwendung eines Antagonisten/Inhibitors nach Anspruch 13 zur Herstellung eines Arzneimittels zur Behandlung von Entzündung, rheumatoider Arthritis, Atherosklerose, Allergie oder infektiösen Krankheiten.

20. Verfahren zum Auffinden eines Antagonisten/Inhibitors oder Agonisten/Aktivators des Polypeptids nach Anspruch 10, umfassend:
(a) Inkontaktbringen des Polypeptids und einer Säugerzelle oder einer Membranpräparation, die einen Rezeptor des Polypeptids exprimiert, mit einem Wirkstoff; und
(b) Messen, ob der Wirkstoff die Wechselwirkung des Polypeptids mit seinem Rezeptor oder die Antwort, die sich aus einer solchen Wechselwirkung ergibt, erhöht oder blockiert.

## Revendications

1. Polynucléotide sélectionné parmi la groupe constitué
(a) de polynucléotides codant pour au moins la forme mature du polypeptide ayant la séquence d'acides aminés déduite comme représenté sur la figure 1,
(b) de polynucléotides ayant la séquence codante comme représenté sur la figure 1 codant pour au moins la forme mature du polypeptide,
(c) de polynucléotides codant pour le polypeptide ayant la séquence d'acides aminés d'au moins la forme mature du polypeptide codé par l'ADNc contenu dans ATCC 75703,
(d) da polynucléotides ayant la séquence codante de l'ADNc contenu dans ATCC 75703 codant pour au moins la forme mature du polypeptide,
(e) de polynucléotides codant pour un fragment d'un polypeptide codé par un quelconque polynucléotide parmi les polynucléotides (a) à (d), ledit fragment étant chimio-attractif pour des leucocytes,
(f) de polynucléotides, dont la séquence est au moins identique à 70 % à la séquence d'un quelconque polynucléotide parmi les polynucléotides (a) à (d) et qui codent pour un polypeptide qui est chimio-attractif pour des leucocytes, et
(g) de polynucléotides dont la séquence est au moins identique à 70 % à la séquence d'un quelconque polynucléotide parmi les polynucléotides (a) à (d) et dont le brin complémentaire s'hybride sous des conditions stringentes à un quelconque polynucléotide tel que défini dans (a) à (d) et qui codent pour un polypeptide qui est chimio-attractif pour des leucocytes,
ou la brin complementaire d'un tel polynucléotide.

2. Polynucléotide selon la revendication 1, dans lequel le polypeptide codé ou un fragment de celui-ci est chimio-attractif pour des basophiles.

3. Polynucléotide selon la revendication 1 ou 2, qui est un ADN ou ARN.

4. ADN selon la revendication 3, qui est un ADN génomique.

5. Vecteur contenant le polynucléotide selon l'une quelconque des revendications 1 à 4.

6. Vecteur selon la revendication 5, dans lequel le polynucléotide est lié de manière opérationnelle à des séquences de commande d'expression permettant l'expression dans des cellules hôtes procaryotes ou eucaryotes.

7. Cellule hôte transformée génétiquement à l'aide du vecteur de la revendication 5 ou 6.

8. Procédé pour produire un polypeptide, qui est chimio-attractif pour des leucocytes, comportant : la culture de la cellule hôte de la revendication 7 et la récupération du polypeptide code par ledit polynucléotide provenant de la culture.

9. Procédé pour produire des cellules capables d'exprimer un polypeptide, qui est chimio-attractif pour des leucocytes, comportant la transformation génétique de cellules à l'aida du vecteur de la revendication 5 ou 6.

10. Polypeptide comportant la séquence d'acides aminés codée par le polynucléotide selon l'une quelconque des revendications 1 à 4, ou pouvant être obtenu par le procédé de la revendication 8.

11. Polypeptide selon la revendication 10, qui comporte un résidu méthionine au niveau de la terminaison N.

12. Anticorps dirigé contre le polypeptide de la revendication 10 ou 11.

13. Antagoniste/inhibiteur dirigé contre le polypeptide de la revendication 10 ou 11, ledit antagoniste/inhibiteur étant une structure d'ADN ou d'ARN anti-sens.

14. Molécule d'acide nucléique qui s'hybride d'une manière spécifique sous des conditions stringentes à un polynucléotide selon l'une quelconque des revendications 1 à 4.

15. Composition pharmaceutique comportant le polynucléotide selon l'une quelconque des revendications 1 à 4, la molécule d'acide nucléique de la revendication 14, un antagoniste/inhibiteur de la revendication 13 ou le polypeptide de la revendication 10 ou 11, et de manière facultative, un excipient pharmaceutiquement acceptable.

16. Composition diagnostique comportant un polynucléotide selon l'une quelconque des revendications 1 à 4, la molécule d'acide nucléique de la revendication 14 ou l'anticorps de la revendication 12.

17. Utilisation d'un polypeptide selon la revendication 10 ou 11, ou d'un polynucléotide selon l'une quelconque des revendications 1 à 4, pour la préparation d'une composition pharmaceutique pour favoriser une cicatrisation de plaie, pour le traitement de tumeurs, d'effusions pleurales ou d'ascites, pour combattre des infections parasites ou pour la régulation d'hématopoïèse.

18. Utilisation selon la revendication 17, dans laquelle l'infection parasita est une schistosomiase, une trichinose ou une ascaridiase.

19. Utilisation de l'antagoniste/inhibiteur de la revendication 13 pour la préparation d'une composition pharmaceutique pour le traitement d'une inflammation, d'une polyarthrite rhumatoïde, d'une athérosclérose, d'une allergie ou de maladies infectieuses.

20. Procédé pour identifier un antagoniste/ inhibiteur ou un agoniste/activateur du polypeptide de la revendication 10, comportant les étapes consistant à :
(a) mettre en contact le polypeptide et une cellule mammifère ou une préparation membranaire exprimant un récepteur du polypeptide avec un médicament, et
(b) mesurer si le médicament accentue ou bloque l'interaction du polypeptide avec son récepteur ou la réponse qui résulte de cette interaction.
